(19) Europäisches Patentamt
European Patent Office
Office européen des brevets

(11) Publication number: 0 017 457 B1

(12) EUROPEAN PATENT SPECIFICATION

(45) Date of publication of patent specification: 26.01.83

(51) Int. Cl.³: C 08 F 8/00, C 07 C 15/107, C 10 M 3/12, C 08 F 6/10

(21) Application number: 80300998.4

(22) Date of filing: 31.03.80

# (54) The use of a synthetic oil as a lubricating oil.

(30) Priority: 05.04.79 US 27226

(43) Date of publication of application: 15.10.80 Bulletin 80/21

(45) Publication of the grant of the patent: 26.01.83 Bulletin 83/4

(84) Designated Contracting States: BE DE FR GB IT NL

(56) References cited:
BE - A - 677 878
GB - A - 1 107 101
US - A - 3 081 288

(73) Proprietor: MOBIL OIL CORPORATION
150 East 42nd Street
New York New York 10017 (US)

(72) Inventor: Brennen, James Aloysious
70 Kings Highway North
Cherry Hill New Jersey (US)
Inventor: Norris, Henry David
449 Sprice Lane
Woodbury New Jersey (US)

(74) Representative: Cooper, John Anthony
Mobil Court 3 Clements Inn
London WC2A 2EB (GB)

EP 0 017 457 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# The use of synthetic oil as a lubricating oil

This invention is concerned with the production of synthetic lubricating oils. One class of high molecular weight synthetic oils which have received much attention for use as a base stock in industrial and automotive oils are the hydrogenated polyolefins. In general, such oils are produced by polymerizing an olefin or mixture of olefins in the presence of an aluminium halide catalyst followed by hydrogenation.

There are, however, at least two serious problems associated with the production of such oils. First, prior to hydrogenation, the polyolefin product has an unacceptably high metal corrosion rate. Second, the polyolefin product brings about a rapid deactivation of the hydrogenation catalyst used in the hydrogenation step. Both of these problems have greatly affected the manufacturing costs of hydrogenized polyolefin oils. For example, the high corrosion rates severely limit air-stream time, while the deactivation necessitates frequent replacement of the hydrogenation catalyst.

It has now been discovered that both of the problems described above are related to organic halide compounds formed during the polymerization reaction. Trace amounts of water present in the polymerization reactor react with the aluminum halide polymerization catalyst to form hydrogen halide and complex aluminum oxyhalide compounds. The aluminum halide catalyst then catalyzes the addition of the hydrogen halide so formed to an olefin. These reactions may be shown as follows:

$$H_2O + AlCl_3 \longrightarrow HCl + AlCl_2OH$$

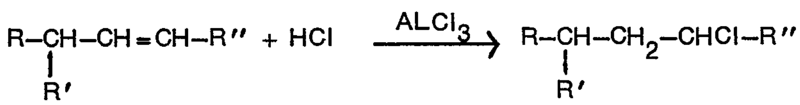


where R, R′ and R″ may be straight or branched hydrocarbyl groups or hydrogen.

The organic halides so formed are of variable stability and may decompose on heating with liberation of hydrogen halide. This has led to corrosion in the distillation section of the plant. The corrosion is attributed to the combination of the liberated hydrogen halide with trace amounts of water in the cooler sections of the recovery system. In addition, organic halides which survive the distillation are carried into the hydrogenation vessel and are catalytically dehydro-halogenated. The hydrogen halide thus formed reacts with and deactivates the hydrogenation catalyst.

The present invention solves both the corrosion and hydrogenation catalyst deactivation problems described herein above. This is accomplished by adding a monocyclic aromatic compound, e.g., benzene, toluene (methylbenzene) or xylene (dimethylbenzene), to the polyolefin reaction product prior to the removal of the aluminum halide catalyst to scavenge the organic halide compounds by alkylation of the aromatic compound. The polyolefin products also alkylate the aromatic so that hydrogenation of the final product is not necessary. The reactions may be illustrated as follows:

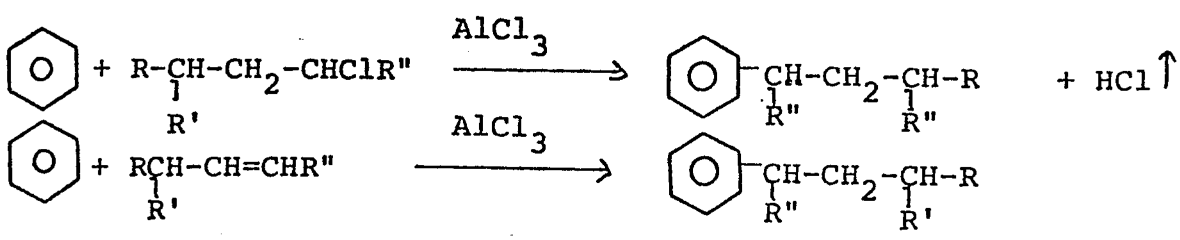


One of the unexpected advantages of the method of this invention was the discovery that the product obtained was equal in value to the hydrogenated polyolefin as a lubricant. It was surprising to find that the products of the present method have similar viscosity, stability, and lubricity properties.

The present invention thus provides for the use of an oil product as a lubricating oil which is obtained by a process for making a high molecular weight oil of lubricating viscosity and which comprises (1) polymerizing a $C_8$—$C_{10}$ olefin in the presence of an aluminum halide catalyst to give a polymer containing from 20 to 150 carbon atoms, preferably 30 to 100 carbon atoms and (2) contacting the total product mixture from the polymerization step, including the aluminum halide catalyst, with a monocyclic aromatic compound under alkylation conditions.

Of particular interest in connection with the present invention is the continuous polymerization, with catalyst recycle, of olefins to liquid polymers useful as synthetic lubricants. A wide variety of olefins can be polymerized with the stable catalyst solution. However, this invention is concerned with those containing only 8 to 10 carbon atoms, i.e. octene, nonene and decene, and mixtures thereof. They

can be straight chain or branched chain. Although preferred among these are the 1-olefins, those having internal double bonds are contemplated. Furthermore, the olefin reactant can be a single olefin or a mixture of olefins, of which the following are examples: octene-1; octene-2; 2-ethylhexene-1; nonene-1; nonene-2, decene-1; and decene-2.

The catalyst used in the present invention may be solid aluminum halide or a solution or complex of an aluminum halide, such as the chloride or bromide, dissolved in an ester, the solution or complex containing more than one mole of the halide per mole of ester. U.S. 3,725,498 and U.S. 3,833,678 describe the solution or complex aluminum halide catalysts in greater detail.

In general, the amount of, for example, aluminum halide dissolved per mole of ester will be between 1.1 moles and 1.4 moles. A 1:1 mole solution has little or no catalytic activity. The aluminum halide in excess of one mole in the solution appears to be the component that imparts catalytic activity to the catalyst solution. Thus, the amount of solution employed to catalyze the reaction will be governed only by the need to provide sufficient excess of aluminum halide to catalyze the olefin polymerization reaction that is ordinarily catalyzed by solid aluminum halide.

The solution of aluminum halide in ester is formed readily. A 1:1 mole solution or complex readily forms at room temperature and this solution is capable of dissolving additional aluminum halide at temperatures of 30—50°C. In order to avoid hydrolysis due to moisture, it is preferred to prepare the catalyst solution in a dry, inert atmosphere, such as nitrogen or dry air.

The solvent ester, in accordance with this invention, is the methyl ester of certain alkanoic acids. The esters contemplated include methyl esters of such acids as n-butyric, n-valeric, n-hexanoic, isovaleric, trimethylacetic, 2-methylvaleric, 2-ethylbutyric, and 2-ethylhexanoic acids.

The polymerization of the olefin is carried out at temperatures of between 0°C and 100°C for a period of time of 1—3 hours. Ordinarily it is carried out at substantially atmospheric pressure, but, particularly with lower olefins, superatmospheric pressures sufficient to maintain liquid phase can be advantageously employed. The amount of catalyst employed will generally be 0.1 to 5 percent, by weight of olefin, based on excess aluminum halide, e.g., the chloride. In some operations, in order to render polymer products less viscous and more readily handled, a solvent inert to the polymerization can be used. Suitable solvents include kerosene and paraffins, such as heptane, octane, isooctane, or decane.

Because the catalyst solution is a heavy liquid, when its removal is desired the effluent from the reactor is permitted to stand quietly until the major amount of the catalyst solution has separated as a lower heavy layer. Alternatively a centrifuge may be used to speed the separation. This layer is recycled, after reconstitution, with fresh aluminum chloride, as needed. Then, the remaining polymer product can be washed free of any residual catalyst solution, dried and freed of solvent and monomer by distillation.

In accordance with the present invention, the polyolefin reaction product is contacted with a monocyclic aromatic compound prior to the removal of the aluminum halide catalyst.

The monocyclic aromatics used in this invention may contain from 0 to 2 $C_1$—$C_{10}$ hydrocarbyl substituents. Obviously, when no substituent is present, the aromatic is benzene. Other hydrocarbyl groups that may be attached include ethyl, butyl, octyl and decyl.

The alkylation reaction is generally carried out at from 80° to 100°C and requires varying times of from 1 to 3 hours. While an amount of aromatic compound equivalent to the polymer and organic halide in the reaction mixture formed may be used, it is preferred to use an excess of aromatic compound corresponding to from 5 to 10 times the amount of polymer plus organic halide present. In addition, in commercial operations it is preferred to add the reaction mixture as obtained, i.e., containing the aluminum halide catalyst, to a hot aromatic compound. This is true even though the illustrations which follow involve adding benzene to the mixture.

Alkylation of an aromatic compound with an alkyl halide is described in greater detail in U.S. 2,506,551. Alkylation of an aromatic compound with a polyolefin is described in greater detail in U.S. 3,600,451 and U.S. 4,035,308. In addition, British Patent No. 1,107,101 discloses a process for preparing an alkyl aromatic hydrocarbon material suitable for producing oil-soluble sulfonates by dimerizing one or more olefins containing from 3 to 18 carbon atoms in the presence of a Friedel-Crafts catalyst, and then alkylating an aromatic hydrocarbon with the resultant dimerized olefin, conveniently by transferring the entire reaction mass from the dimerization step to the alkylation step. It is, however, to be appreciated that none of these patents disclose or suggest the problems associated with the production of high molecular weight lubricants by polymerization and hydrogenation described hereinabove. Nor do these patents suggest that an oil having similar properties to the hydrogenated polyolefins can be prepared by contacting the total product of the polymerization reaction, including the aluminum halide catalyst, with a monocyclic aromatic compound at alkylation conditions.

### Example 1 — Polymerization

The catalyst was prepared by dissolving anhydrous aluminum chloride in methyl-n-butyrate at room temperature, in a molar proportion, respectively, of 1.31:1. This catalyst and 1-decene were metered at 38 g./hr. and 552 g./hr., respectively, over a two-hour period into a reaction vessel fitted with stirrer, thermometer and reflux condenser. The mixture was stirred and maintained at 45°C. After addition of the catalyst and 1-decene was complete, the mixture was maintained at 45°C for an

additional 15 minutes. The reaction mixture was then quenched with 250 ml of an 8% aqueous solution of HCl, was made basic with dilute $NH_4OH$ and was washed out with water until neutral. Olefin monomer and dimer were removed by distillation, the monomer at 80°C and 8000 Pa of pressure and the dimer at 120°C and 133.3 Pa of pressure. The yield of oil, based on olefin, was 94%. The oil had a viscosity of $36.6 \times 10^{-6} m^2/s$ at 99°C and $374 \times 10^{-6} m^2/s$ at 38°C, and contained 170 ppm chlorine.

Example 2 — Polymerization followed by alkylation

The catalyst was prepared by dissolving anhydrous aluminum chloride in methyl-n-butyrate at room temperature, in a molar proportion, respectively, of 1.31:1.

This catalyst and 1-decene were metered at 38 g./hr. and 552 g./hr., respectively, over a two-hour period into a reaction vessel fitted with stirrer, thermometer and reflux condenser. The mixture was stirred and maintained at 45°C. After addition of the catalyst and 1-decene was complete, the mixture was maintained at 45°C for an additional 15 minutes. 1000 ml. of benzene was then added and the mixture was stirred at reflux (85—88°C) for 2.5 hours, after which the reaction mixture was quenched with 250 ml. of 8% aqueous HCL, was made basic with dilute $NH_4OH$ and was washed out with water until neutral. Benzene, olefin monomer and dimer were removed by distillation, the monomer at 80°C and 8000 Pa of pressure and the dimer at 120°C and 133.3 Pa of pressure. The yield of oil, based on olefin, was 95%. The oil had a number average molecular weight of about 1400, a viscosity of $43.8 \times 10^{-6} m^2/s$ at 99°C and $521 \times 10^{-6} m^2/s$ at 38°C, and contained only 5 ppm. of chlorine. Infra red spectra of the oil showed the presence of aromatic rings with only a trace of olefin.

Example 3

A polymerization followed by alkylation identical to Example 2 was done, except that 940 g. of 15/85 octene/decene was substituted for the decene, the addition time was shortened to one hour and 42 minutes and a 150 ml. portion of the reaction mixture was withdrawn at the end of the 15 min. hold period prior to the addition of the benzene.

The 150 ml. polymerization product was contacted with aqueous HCl, made basic with dilute $NH_4OH$ and washed with water as described above. Olefin monomer and dimer were removed by distillation. From this 150 ml. portion there was obtained a 99% yield of an oil with a viscosity of $38.2 \times 10^{-6} m^2/s$ and $400 \times 10^{-6} m^2/s$ at 99°C and 38°C, respectively. The oil contained 174 ppm. of chlorine.

The remainder of the polymerization product was alkylated, washed and distilled as described in Example 2.

The alkylation product, obtained in >93% yield, had a number average molecular weight of 1467, a viscosity of $46.4 \times 10^{-6} m^2/s$ at 99°C and $550 \times 10^{-6} m^2/s$ at 38°C and contained <1 ppm. of chlorine.

Example 4

A polymerization and an alkylation reaction were performed as in Example 2, except that 867 g. of toluene was substituted for the benzene and the mixture was heated to 85°C and held at this temperature for 2.5 hours. The product oil (95% yield) had a viscosity of $43.6 \times 10^{-6} m^2/s$ at 99°C and $529 \times 10^{-6} m^2/s$ at 38°C and contained 4 ppm. of chlorine.

Example 5

In order to show that alkylation is necessary and that heating alone will not produce products with low chlorine content, the following experiment was made. The procedure of Example 2 was followed, except that 684 g. of n-heptane was substituted for the benzene and the mixture was heated to 100°C. The oil obtained had a viscosity of $38.8 \times 10^{-6} m^2/s$ at 99°C and $422 \times 10^{-6} m^2/s$ at 38°C and contained 109 ppm. chlorine.

Example 6

The polyolefin oil of Example 1 (177 g) was hydrogenated at 175°C and 500 psig $H_2$ in the presence of 5.3 g. of nickel a kieselguhr catalyst. The product had a viscosity of $37.5 \times 10^{-6} m^2/s$ at 99°C and $391 \times 10^{-6} m^2/s$ at 38°C and contained 4 ppm. chlorine. This example illustrates that the oils produced by the process of this invention have properties similar to the hydrogenated polyolefin oils.

**Claims**

1. The use of an oil product as a lubricating oil, the product being obtained by a process which comprises (1) polymerizing a $C_8$—$C_{10}$ olefin in the presence of an aluminum halide catalyst to give a polymer containing from 20 to 150 carbon atoms and (2) contacting the total product mixture from the polymerization step, including the aluminum halide catalyst with a monocyclic aromatic compound under alkylation conditions.

2. The use of an oil product as a lubricating oil, the product being as claimed in claim 1 wherein said polymer contains from 30 to 100 carbon atoms.

3. The use of an oil product as a lubricating oil, the product being as claimed in claim 1 or claim 2

wherein said aromatic compound is benzene, toluene or a substituted aromatic compound with up to two $C_1$—$C_{10}$ hydrocarbyl substituents.

4. The use of an oil product as a lubricating oil, the product being as claimed in any preceding claim wherein said olefin is 1-decene or a mixture of 1-octene and 1-decene.

**Revendications**

1. L'emploi d'un produit huileux comme huile lubrifiante, le produit étant obtenu selon un procédé qui comprend:

(1) la polymérisation d'une oléfine en $C_8$—$C_{10}$ en présence d'un catalyseur constitué d'halogénure d'aluminium pour former un polymère contenant 20 à 150 atomes de carbone; et

(2) le contact du mélange produit total du stade de polymérisation, y compris le catalyseur constitué d'halogénure d'aluminium, avec un composé aromatique monocyclique dans les conditions d'alkylation.

2. L'emploi d'un produit huileux comme huile lubrifiante, le produit étant comme revendiqué dans la revendication 1, où ledit polymère contient 30 à 100 atomes de carbone.

3. L'emploi d'un produit huileux comme huile lubrifiante, le produit étant comme revendiqué dans la revendication 1 ou la revendication 2, où ledit composé aromatique est le benzène, le toluène ou un composé aromatique substitué avec jusqu'à deux substituants hydrocarbyles en $C_1$—$C_{10}$.

4. L'emploi d'un produit huileux comme huile lubrifiante, le produit étant comme revendiqué dans l'une quelconque des revendications précédentes, où ladite oléfine est le décène-1 ou un mélange d'octène-1 et de décène-1.

**Patentansprüche**

1. Die Verwendung eines Ölprodukts als ein Schmieröl, wobei das Produkt durch ein Verfahren erhalten wird, das umfaßt (1) Polymerisieren eines $C_8$—$C_{10}$-Olefins in Gegenwart eines Aluminiumhalogenid-Katalysators, das ein Polymer ergibt, das von 20 bis 150 Kohlenstoffatome enthält und (2) Kontaktieren der gesamten Produktmischung aus der Polymerisationsstufe, einschließlich des Aluminiumhalogenid-Katalysators, mit einer monocyclischen aromatischen Verbindung unter Alkylierungsbedingungen.

2. Die Verwendung eines Ölprodukts als ein Schmieröl, wobei das Produkt eines ist, wie es in Anspruch 1 beansprucht wurde, wobei das genannte Polymer von 30 bis 100 Kohlenstoffatome enthält.

3. Die Verwendung eines Ölprodukts als ein Schmieröl, wobei das Produkt eines ist, wie es in Anspruch 1 oder Anspruch 2 beansprucht wurde, wobei die genannte aromatische Verbindung Benzol, Toluol oder eine substituierte aromatische Verbindung mit bis zu zwei $C_1$—$C_{10}$ Kohlenwasserstoffsubstituenten ist.

4. Die Verwendung eines Ölprodukts als ein Schmieröl, wobei das Produkt eines ist, wie es in einem der vorangehenden Ansprüche beansprucht wurde, wobei das genannte Olefin 1-Decen oder eine Mischung aus 1-Octen und 1-Decen ist.